# EUROPÄISCHE PATENTSCHRIFT

(11) **EP 4 171 728 B1**
(45) Veröffentlichungstag und Bekanntmachung des Hinweises auf die Patenterteilung: **18.09.2024**
(21) Anmeldenummer: 21731480.6
(22) Anmeldetag: 09.06.2021
(51) Int. Cl.: A61N 1/375

(54) **MEDIZINISCHES HANDHABUNGSSET**
MEDICAL HANDLING KIT
KIT D'ASSISTANCE MÉDICALE

(30) Priorität: 30.06.2020 DE 102020117142
(43) Veröffentlichungstag der Anmeldung: 03.05.2023
(73) Patentinhaber: Neuroloop GmbH, 79110 Freiburg (DE)
(72) Erfinder: KEMPTER, Daniel, 79110 Freiburg (DE); KIMMIG, Fabian, 79110 Freiburg (DE); BORETIUS, Tim, 79110 Freiburg (DE)
(74) Vertreter: Rösler, Uwe
(86) Internationale Anmeldenummer: PCT/EP2021/065423
(87) Internationale Veröffentlichungsnummer: WO 2022/002543

(56) Entgegenhaltungen:
- DE-A1- 102018 213 120
- US-A- 4 971 057
- US-A- 5 050 602
- US-A1- 2004 230 267
- US-A1- 2006 030 918
- US-A1- 2008 255 630
- US-A1- 2011 270 068

## Beschreibung

### Technisches Gebiet

Die Erfindung bezieht sich auf ein medizinisches Handhabungsset zur Konnektierung zweier räumlich getrennt voneinander angeordneter Implantate über eine flexible Verbindungsleitung, längs der eine mechanische Schnittstelle nach Art einer SteckerBuchsen-Verbindung vorgesehen ist

### Stand der Technik

Implantierbare medizinische Vorrichtungen zum Zwecke der elektrischen Stimulation lokaler intrakorporaler Bereiche, kurz implantierbare Pulsgeber (IPG) beispielsweise für herztherapeutische Defibrillations-, Schrittmacher- sowie Resynchronisationsanwendungen, für neurostimulationstherapeutische Maßnahmen, wie bspw. Rückenmarkstimulation, Hirnstimulation oder Vagusnervstimulation, um nur einige zu nennen, weisen in aller Regel ein in sich fluiddicht abgeschlossenes Gehäuse auf, in dem Komponenten zur elektrischen Pulsgeneration enthalten sind, so zumindest eine elektrische Energiequelle, in Form einer Batterie oder einer Induktionsspule, und eine mit dieser verbundenen elektrischen Schaltungsstruktur. Zudem schließt zumeist an das Gehäuse ein sogenanntes Kopfteil an, in dem eine mit der Energiequelle bzw. der elektrischen Schaltungsstruktur elektrisch verbundene, elektrische Kontaktanordnung enthalten ist, in die eine fluiddicht mit dem Kopfteil abschließende Steckeranordnung einfügbar ist, die über elektrische Zu- und Ableitungen, die typischerweise jeweils zur einer flexiblen Verbindungsleitung zusammengefasst sind, mit wenigstens einer separaten intrakorporalen Elektrodenanordnung zum Zwecke einer lokalen Applikation elektrischer Stimulationssignale sowie einer Ableitung intrakorporal lokal abgegriffener elektrischer Signale zur Gehäuse-seitig vorhandenen elektrischen Schaltungsstruktur, elektrisch verbunden ist.

In der Druckschrift EP 3 204 105 B1 ist eine implantierbare Elektrodenanordnung offenbart, die zu Zwecken der elektrischen Stimulation selektierter Nervenfasern längs des Vagusnerv in Form einer Cuff-Elektrodenanordnung manschettenartig lokal um den Vagusnerv im Halsbereich angelegt wird. Demgegenüber befindet sich das Implantatgehäuse, kurz IPG (implantierbarer Pulsgenerator), das sämtliche für den Betrieb und die Funktion der elektrischen Stimulation erforderlichen Komponenten umfasst in einem chirurgisch leicht zugänglichen Bereich, vorzugsweise an einer Stelle unterhalb des Schlüsselbeins.

US 2006/030918 A1 offenbart einen Konnektor, z. B. für den Operationssaal, in einem System zur Simulation des Rückenmarks mit mehreren Elektroden, dessen Mandrinendraht mit Hilfe von Klebstoff dauerhaft am Mandringriff befestigt ist und der durch ein zentrales Lumen in das Leitungssystem eingreift

Zur Zwecken der Implantation sowohl der Cuff-Elektrodenanordnung als auch des IPGs werden zwei Hautschnitte vorgenommen, von denen einer im Brustbereich zur Verortung des IPGs und der andere am Halsbereich zur Anbringung der Cuff-Elektrodenanordnung längs des Vagusnervs dienen. Typischerweise ist die Cuff-Elektrodenanordnung einseitig fest mit einer flexibel ausgebildeten elektrischen Verbindungsleitung versehen, an deren der Cuff-Elektrodenanordnung gegenüberliegenden Leitungsende eine elektrische Schnittstelle, vorzugsweise in Form eines multipoligen Steckerteils angeordnet ist, den es gilt, in ein am IPG vorgesehenes Buchsenteil zu fügen.

Um die durch diesen chirurgischen Eingriff für den Patienten verbundene Belastung möglichst gering zu halten besteht der Bedarf nach einer möglichst schonenden intrakorporalen Verlegung der flexiblen, elektrischen Verbindungsleitung zwischen IPG und der Cuff-Elektrodenanordnung. Der hierfür erforderliche Eingriff soll für den Operateur zudem in einer möglichst kurzen Zeit sicher und fehlerfrei vollzogen werden können.

Die Druckschrift US 2013/0324994 A1 offenbart Systeme zur Modulation von Nerven in Knochen, die im Wesentlichen einen Tubus und einen darin innengeführten Mandrin vorsehen, der eine fest am distalen Mandrinbereich angebrachte Mandrinspitze aufweist, der das distale Tubusende überragt.

### Darstellung der Erfindung

Der Erfindung liegt die Aufgabe zu Grunde ein medizinisches Handhabungsset zur Konnektierung zweier räumlich getrennt voneinander angeordneter Implantate über eine flexible Verbindungsleitung, längs der eine mechanische Schnittstelle nach Art einer Stecker-Buchsenverbindung vorgesehen ist, derart anzugeben, so dass die zwei an unterschiedlichen intrakorporalen Bereichen verorteten Implantate patientenschonend, d.h. ohne oder weitgehend ohne dermatologische Irritationen, über die elektrische Verbindungsleitung verbunden werden sollen. Die hierfür erforderlichen medizinischen Hilfsmittel sollen überdies dem Operateur eine intuitiv leichte, fehlerfreie und schnell durchführbare Handhabung ermöglichen.

Die Lösung der der Erfindung zu Grunde liegenden Aufgabe ist im Anspruch 1 angegeben. Den Erfindungsgedanken in vorteilhafter Weise weiterbildende Merkmale sind Gegenstand der Unteransprüche sowie insbesondere der weiteren Beschreibung unter Bezugnahme auf ein Ausführungsbeispiel zu nehmen.

Das lösungsgemäße medizinische Handhabungsset zur Konnektierung zweier räumlich getrennt voneinander angeordneter Implantate über eine flexible elektrische Verbindungsleitung, längs der eine mechanische Schnittstelle nach Art einer SteckerBuchsen-Verbindung vorgesehen ist, umfasst einen Tubus sowie einen darin geführten Mandrin, der eine über eine erste Fügeverbindung lösbar fest am distalen Mandrinbereich angebrachte Mandrinspitze vorsieht, die das distale Tubusende im gefügten Zustand überragt.

Vorzugsweise schließt die Mandrinspitze bündig, vorzugsweise fluiddicht am distalen Tubusende an, um so ein Eindringen von Körperflüssigkeit, insbesondere Blut, in das Lumen des Tubus während eines Gewebepenetrationsvorganges zu vermeiden.

Die Mandrinspitze ist über eine lösbar feste, erste Fügeverbindung am distalen Mandrinbereich angebracht. Die erste Fügeverbindung ist vorzugsweise in Form einer Gewinde-Gegengewinde-Verbindung oder in Art eines Bajonettverschlusses ausgebildet, um einerseits einen festen Sitz der Mandrinspitze am Mandrin während des operativen Eingriffes zu gewährleisten und andererseits dem Operateur eine unkomplizierte und schnelle Entnahme der Mandrinspitze vom distalen Mandrinbereich zu ermöglichen.

Der distale Mandrinbereich weist überdies eine erste Fügekontur auf, die Teil einer zweiten Fügeverbindung ist, die sich vorzugsweise in Art und Ausgestaltung von der ersten Fügeverbindung unterscheidet.

Das lösungsgemäße medizinische Handhabungsset weist überdies ein separates Adapterelement auf, das eine zur ersten Fügekontur gegenkonturiert ausgebildete Gegenfügekontur zur Ausbildung der zweiten Fügeverbindung besitzt. Zudem weist das Adapterelement eine zweite Fügekontur auf, die zum Stecker- oder Buchsenteil der mechanischen Schnittstelle gegenkonturiert ausgebildet ist und geeignet zur Ausbildung einer dritten Fügeverbindung ist. Im Unterschied zur ersten Fügeverbindung zwischen der Mandrinspitze und dem distalen Mandrinbereich sind die zweite und dritte Fügeverbindung in Art und Ausbildung vergleichbar ausgebildet und basieren vorzugsweise auf einem Schnapp- und/oder Rastmechanismus.

In einem bevorzugten Ausführungsbeispiel weist die erste Fügekontur am distalen Mandrinbereich eine Kopf-förmige Struktur auf, die in die konkav ausgebildete, am Adapterelement vorgesehene Gegenfügekontur unter Erzeugung eines akustisch wahrnehmbaren Schnappgeräusches presskraft-unterstützt fügbar ist. Alternativ ist es möglich die am distalen Mandrinbereich vorgesehene erste Fügekontur konkav auszubilden, in die eine entsprechend gegenkonturiert ausgebildete kopf-förmige Struktur seitens des Adapterelementes presskraft-unterstützt fügbar ist. Die akustische Wahrnehmung beruht auf einer impulsartigen Fügekollision, die durch eine Art Aufpralleffekt zwischen den beiden Fügepartnern hervorgerufen wird und die auch nur bei einer erfolgreichen Fügung erzeugt wird, so dass das wahrnehmbare Fügegeräusch für den Operateur als Bestätigungssignal für eine erfolgreiche Fügung gewertet werden kann. Diese Art der akustischen Rückbestätigung ist für den Operateur insofern wertvoll, zumal er aufgrund der Kleinteiligkeit und der beengten Platzverhältnisse während der Operationsdurchführung nicht immer Blickkontakt zu den einzelnen Instrumenten hat.

Die am Adapterelement angebrachte zweite Fügekontur ist wenigstens teilweise gegenkonturiert zum endseitig an der flexiblen Verbindungsleitung angebrachten Stecker- oder Buchsenteil ausgebildet, so dass es möglich ist das Stecker- oder Buchsenteil einseitig auf die am Adapterelement angebrachte zweite Fügekontur einzulegen und mit Hilfe eines Rast- oder Schnappmechanismus axial- und drehfest am Adapterelement lösbar fest zu sichern. Die am Adapterelement hierzu vorgesehene zweite Fügekontur ist speziell an die Ausbildung des Stecker- oder Buchsenteils angepasst. Alternativ ist die zweite Fügekontur im Falle eines endseitig an der flexiblen Verbindungsleitung angebrachten Steckerteils in Art einer Sacklochanordnung ausgebildet, in die das zapfenartig ausgebildete Steckerteil lösbar fest fügbar ist. Im Falle eines an der flexiblen Verbindungsleitung angebrachten Buchsenteils ist die am Adapterelement angebrachte zweite Fügekontur steckerartig zum lösbar festen Einfügen in das Buchsenteil ausgebildet.

Zur Sicherung dieser sich zwischen dem Adapterelement und dem an der flexiblen Verbindungsleitung angebrachten Stecker- oder Buchsenteil ausbildenden dritten Fügeverbindung weist die zweite Fügekontur wenigsten eine Klemmbacke auf, die form- und oder kraftschlüssig an einer am Stecker- oder Buchsenteil angebrachte Außenkontur zur Anlage kommt.

Vorzugsweise ist das Adapterelement zylinderförmig ausgebildet, das an seinen zwei sich längs der Zylinderachse gegenüberliegenden Zylinderendbereichen einerseits die Gegenfügekontur und andererseits die zweite Fügekontur aufweist. Zudem weist das Adapterelement sowie die als Stecker- oder Buchsenteil ausgebildete Fügekontur der flexiblen Verbindungsleitung in gefügten Zustand einen maximalen Querschnittsdurchmesser auf, der kleiner dimensioniert ist als ein dem Tubus zugeordneter Querschnitt, so dass das Adapterelement unter Ausbildung der zweiten und dritten Fügeverbindung axial vollständig in und durch den Tubus gezogen werden kann.

Ein bevorzugtes Ausführungsbeispiel ist in den nachstehenden Figuren illustriert.

### Kurze Beschreibung der Erfindung

Die Erfindung wird nachstehend ohne Beschränkung des allgemeinen Erfindungsgedankens anhand von Ausführungsbeispielen unter Bezugnahme auf die Zeichnungen exemplarisch beschrieben. Es zeigen:
- Fig. 1: Tubus mit innengeführten Mandrin und distaler Mandrinspitze,
- Fig. 2: Ansicht eines Adapterelementes,
- Fig. 3: Adapterelement mit eingelegtem Steckerteil, das endseitig an der flexiblen Verbindungsleitung angebracht ist sowie
- Fig. 4: Tubus mit innengeführten Mandrin an dessen Mandrinspitze das Adapterelement angebracht ist

### Wege zur Ausführung der Erfindung, gewerbliche Verwendbarkeit

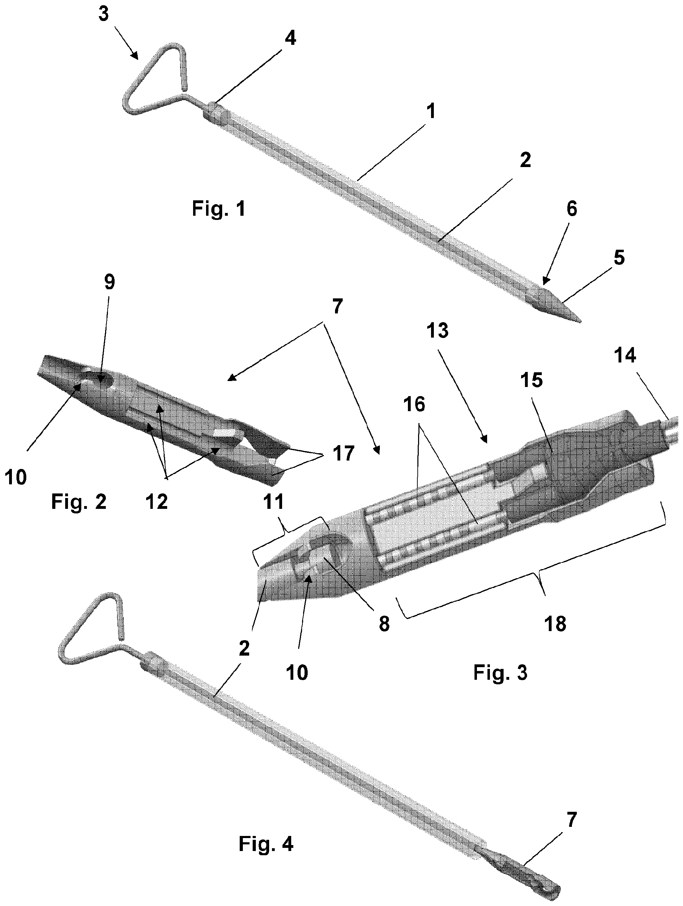

Figur 1 zeigt einen als Hohlkanüle ausgebildeten Tubus 1, in dessen Inneren ein Mandrin 2 gelagert ist, dessen proximales Ende Handgriff-artig zur manuellen Betätigung geformt ist und einem Operateur die Möglichkeit bietet längs des Mandrins 2 sowie des Tubus 1 axiale Zug- und Schubkräfte sowie auch um die Mandrin-Längsachse orientierte Drehmomente dosiert auszuüben.

Das proximale Ende des Tubus 1 sitzt lösbar fest an einem am Mandrin 2 angebrachten mechanischen Anschlag 4 auf, durch den die auf den Mandrin 2 wirkenden Schubkräfte auf den Tubus 1 übertragen werden. Der Mandrin 2 ist an seinem distalen Mandrinbereich mit einer Mandrinspitze 5 über eine erste Fügeverbindung lösbar fest verbunden. Die erste Fügeverbindung 6 besteht vorzugsweise aus einer Gewinde-Gegengewinde-Verbindung oder aus einer Bajonettverbindung, die die Mandrinspitze 5 lösbar fest mit dem distalen Mandrinbereich des Mandrins 2 fügt. Beispielsweise weist hierzu der Mandrin 1 an seinem distalen Mandrinbereich ein Außengewinde auf, das in Eingriff mit einem Innengewinde der Mandrinspitze 5 gebracht wird.

Das in Figur 1 illustrierte medizinische Instrument, bestehend aus einem Tubus 1 und einem innen geführten Mandrin 2, dessen Mandrinspitze 5 fluiddicht am distalen Tubusende anliegt, dient der intrakorporalen Kannülierung, d.h. der Einbringung eines Durchstoßkanals durch einen intrakorporalen Gewebebereich. In Zusammenhang mit der Eingangs erläuterten Implantation der Cuff-Elektrodenanordnung und dem IPG wiurd das in Figur 1 dargestellte medizinische Instrument von Seiten des Brustbereiches in Richtung der implantierten Cuff-Elektrodenanordnung möglichst gewebeschonend nahe unter der Hautoberfläche tunneliert, zur Herstellung eines Verbindungskanals, längs dem die elektrische Verbindungsleitung zwischen Cuff-Elektrodenanordnung und IPG verlaufen soll.

Nach vorgenommener Tunnelierung ragt die Mandrinspitze 5 aus einer im Bereich des Vagusnervs eingebrachten korporalen Öffnung nach außen, so dass der Operateur die Mandrinspitze 5 durch Lösen der ersten Fügeverbindung vom distalen Mandrinbereich separieren kann. Nachfolgend wird ein in Figur 2 und 3 illustriertes Adapterelement 7 an den distalen Mandrinbereich gefügt.

Hierzu weist der Mandrin 2 distalseitig eine erste Fügekontur 8 auf, siehe Figur 3, die kopfartig ausgebildet ist. Demgegenüber sieht das Adapterelement 7 einseitig zur ersten Fügekontur 8 eine gegenkonturiert ausgebildete Gegenfügekontur 9 vor, die im Falle der in Figur 2 und 3 dargestellten Ausbildung in Form einer konkaven Ausnehmung ausgebildet ist. Die Gegenfügekontur 9 weist einen flaschenhalsförmig ausgebildeten Schapp- bzw. Rastmechanismus 10 auf, der dafür sorgt, dass die erste Fügekontur 8 des Mandrins 2 nach Presskraft-beaufschlagtem Fügen in die Gegenfügekontur 9 axial-fest mit dem Adapterelement 7 verbunden ist. Die so hergestellte zweite Fügeverbindung 11 ermöglicht eine Schub- und Zugkraftübertragung zwischen Mandrin 2 und dem Adapterelement 7. Nicht notwendigerweise ist die zweite Fügeverbindung 11 drehfest um die Mandrinlängsachse ausgebildet.

Der Schnapp-/Rastmechanismus 10 ist zudem derart konfektioniert, dass eine bestimmte Presskraft-beaufschlagte Fügekraft überwunden werden muss, um die erste Fügekontur 8 in die Gegenfügekontur 9 überführen zu können. Der Fügevorgang erfolgt schlagartig, d. h. die kopfartig ausgebildete erste Fügekontur 8 schlägt regelrecht in die konkave Ausnehmung der Gegenfügekontur 9 auf, wodurch ein für den Operateur wahrnehmbares Schnapp- bzw. Rastgeräusch entsteht. Dieses dient zur Quittierung bzw. Kontrolle für eine vollständige und sichere Herstellung der zweiten Fügeverbindung 11.

Zudem weist das Adapterelement 7, das vorzugsweise aus einem zylinderförmigen Grundkörper geformt ist, eine zweite Fügekontur 12 auf, die individuell an das Steckerteil 13 angepasst ist, das jeweils endseitig mit der flexiblen Verbindungsleitung 14 verbunden ist. Der in Figur 3 dargestellte und an der elastischen Verbindungsleitung 14 angebrachte Steckerteil 13 sieht einen Steckerkorpus 15 sowie zwei davon abgehende elektrische Kontaktpins 16 vor. Entsprechend der geometrischen Ausbildung des Steckerteils 13 weist die am Adapterelement 7 eingebrachte zweite Fügekontur 12 eine entsprechende Ausnehmung für den Steckerkorpus 15 als auch für beide Kontaktpins 16 auf.

Zwei am Adapterelement 7 angebrachte Klemmbacken 17 halten das Steckerteil 13 in einer raumfesten Position relativ zum Adapterelement 7. Auch in diesem Fall bedarf es der Überwindung einer Mindestfügepresskraft, die dazu führt, dass bei der Herstellung der in Figur 3 dargestellten dritten Fügeverbindung 18 ein für den Operateur akustisch wahrnehmbares Schnapp- bzw. Rastgeräusch erzeugt wird.

Auch das für den Arzt als sicherer Hinweis darauf deint, dass das Steckerteil 13 korrekt und sicher im Adapterteil 7 gefügt ist.

Die Ausbildung der ersten und zweiten Fügeverbindung 11, 18 in Form einer Rast- bzw. Schnappverbindung ermöglicht eine unkomplizierte und schnelle Handhabung für den jeweiligen Arzt, die zudem ein akustisch wahrnehmbar Geräusch erzeugt.

In Figur 4 ist ein Zustand des medizinischen Instrumentes gezeigt, bei dem am distalen Mandrinbereich das Adapterelement 7 gefügt ist, in dem das Steckerteil 13 gefügt ist (nicht dargestellt). Der Mandrin 2 wird relativ zum ortsfest verbleibenden Tubus 1 proximalwärts durch den Tubus 1 gezogen, wodurch die mit dem Steckerteil 13 verbundene elastische Verbindungsleitung 14 durch den Tubus 1 proximalwärts gezogen wird. Sobald das Adapterelement 7 am proximalen Ende des Tubus 1 herausragt, wird der Steckerteil 13 leichtgängig vom Adapterelement 7 gelöst und in die vorzugsweise am IPG vorgesehene Buchse gefügt.

### Bezugszeichenliste

- 1: Tubus
- 2: Mandrin
- 3: Handgriff, proximales Ende des Mandrins
- 4: mechanischer Anschlag
- 5: Mandrinspitze
- 6: erste Fügeverbindung
- 7: Adapterelement
- 8: erste Fügekontur
- 9: Gegenfügekontur
- 10: Schnapp-/Rastmechanismus
- 11: zweite Fügeverbindung
- 12: zweite Fügekontur
- 13: Steckerteil
- 14: elastische Verbindungsleitung
- 15: Steckerkorpus
- 16: Kontaktpins
- 17: Klemmbacken
- 18: dritte Fügeverbindung

## Patentansprüche

1. Medizinisches Handhabungsset zur Konnektierung zweier räumlich getrennt voneinander angeordneter Implantate über eine flexible Verbindungsleitung (14), längs der eine mechanische Schnittstelle nach Art einer Stecker-BuchsenVerbindung vorgesehen ist, mit
- einem Tubus (1) und einem darin innengeführten Mandrin (2), der eine über eine erste Fügeverbindung (6) lösbar fest am distalen Mandrinbereich angebrachte Mandrinspitze (5) vorsieht, die das distale Tubusende im gefügten Zustand überragt,
- einer am distalen Mandrinbereich angebrachten ersten Fügekontur(8), die Teil einer zweiten Fügeverbindung (11) ist, sowie
- einem Adapterelement (7) mit einer zur ersten Fügekontur (8) gegenkonturiert ausgebildeten Gegenfügekontur (9) zur Ausbildung der zweiten Fügeverbindung (11) sowie mit einer zum Stecker- oder Buchsenteil der mechanischen Schnittstelle gegenkonturiert ausgebildeten zweiten Fügekontur (12) die Teil einer dritten Fügeverbindung (18) ist.

2. Handhabungsset nach Anspruch 1,
**dadurch gekennzeichnet, dass** die erste Fügeverbindung (6) in Art einer Gewinde-Gegengewinde-Verbindung oder in Art eines Bajonett-Verschlusses ausgebildet ist.

3. Handhabungsset nach Anspruch 1 oder 2,
**dadurch gekennzeichnet, dass** die zweite und dritte Fügeverbindung (11, 18) auf einem Schnapp- und/oder Rastmechanismus basiert, bei dessen Fügung ein akustisch wahrnehmbares Geräusch entsteht.

4. Handhabungsset nach einem der Ansprüche 1 bis 3,
**dadurch gekennzeichnet, dass** die erste Fügekontur (8) Kopf-förmig und die Gegenfügekontur konkav derart ausgebildet sind, so dass die erste Fügekontur (8) Presskraft-unterstützt in die konkave Gegenfügekontur (9) im Wege eines akustisch wahrnehmbaren Schnappvorganges fügbar ist, oder umgekehrt.

5. Handhabungsset nach einem der Ansprüche 1 bis 4,
**dadurch gekennzeichnet, dass** die flexible Verbindungsleitung (14) wenigstens ein Leitungsende mit einer als Stecker- oder Buchsenteil ausgebildeten Fügekontur aufweist, und
dass die als Stecker- oder Buchsenteil ausgebildete Fügekontur in einer räumlich unveränderlichen Lage relativ zum Adapterelement (7) mit der zweiten Fügekontur (12) unter Ausbildung der dritten Fügeverbindung (18) fügbar ist.

6. Handhabungsset nach einem der Ansprüche 1 bis 5,
**dadurch gekennzeichnet, dass** das Adapterelement (7) sowie die als Stecker- oder Buchsenteil ausgebildete Fügekontur der flexiblen Verbindungsleitung (14) im gefügten Zustand einen maximalen Querschnittdurchmesser aufweisen, der kleiner ist als ein dem Tubus (1) zugeordneter Querschnitt derart, so dass das Adapterelement (7) unter Ausbildung der zweiten und dritten Fügeverbindung (11, 18) axial vollständig innerhalb des Tubus (1) platzierbar ist.

7. Handhabungssetz nach einem der Ansprüche 1 bis 6,
**dadurch gekennzeichnet, dass** das Adapterelement (7) in Art eines Zylinders mit einer Zylinderachse und zwei sich längs der Zylinderachse gegenüberliegenden Zylinderendbereichen ausgebildet ist,
dass die Gegenfügekontur (9) sowie die zweite Fügekontur (12) des Adapterelementes (7) jeweils an den sich gegenüberliegenden Zylinderendbereichen angebracht sind.

8. Handhabungsset nach einem der Ansprüche 1 bis 7,
**dadurch gekennzeichnet, dass** die zweite Fügekontur (12) wenigstens zwei Klemmbacken (17) aufweist, die form- und/oder kraftschlüssig eine Außenkontur des Stecker- oder Buchsenteils im Zustand der dritten Fügeverbindung (18) berühren.

## Claims

1. Medical handling set for connecting two implants, which are arranged spatially separated from each other, by means a flexible connecting line (14), along which a mechanical interface in the form of a plug-socket connection is provided, with
- a tube (1) as well as, guided therein, a mandrin (2) which provides a mandrin tip (5) which is fixedly attached to the distal mandrin region in a releasable manner by means of a first joint (6) and in the joined state protrudes beyond the distal tube end,
- a first joining contour (8) with is applied at the distal mandrin region and forms part of a second joint (11), as well as
- for forming the second joint (11), an adapter element (7) with a counter joining contour (9) which is contoured counter to the first joining contour (8), and with a second joining contour (12) which is contoured counter to the plug or socket part of the mechanical interface and is part of a third joint (18).

2. Handling set according to claim 1,
**characterised in that** the first joint (6) is designed in the form of a thread-counterthread connection or in the form of a bayonet closure.

3. Handling set according to claim 1 or 2,
**characterised in that** the second and third joint (11, 18) are based on a snap-in and/or locking mechanism, during the joining of which an acoustically perceivable sound is produced.

4. Handling set according to any one of claims 1 to 3,
**characterised in that** the first joining contour (8) is head-shaped and the counter joining contour is concave in design, so that in a pressing force-assisted manner, the first joining contour (8) can be fitted into the concave counter joining contour (9) by means of an acoustically perceivable snap-in procedure, or vice-versa.

5. Handling set according to any one of claims 1 to 4,
**characterised in that** the flexible connection line (14) has at least one line end with a joining contour configured as a socket or plug part, and
**in that** the joining contour in the form of the socket or plug part, can, in a spatially invariable position relative to the adapter element (7) be joined to the second joining contour (12), thereby forming the third joint (18).

6. Handling set according to any of claims 1 to 5,
**characterised in that** in the joined state, the adapter element (7) as well as the joining contour of the flexible connecting line (14) designed as a plug or socket part, have a maximum cross-sectional diameter which is smaller than a diameter assigned to the tube (1), so that the adapter element (7) can be axially fully placed within the tube (1), forming the second and third joint (11, 18).

7. Handling set according to any of claims 1 to 6,
**characterised in that** the adapter element (7) is designed in the form of a cylinder with a cylinder axis and two cylinder end regions opposite each other along the cylinder axis,
**in that** the counter joining contour (9) as well as the second joint (12) of the adapter element (7) are respectively provided on opposite cylinder end regions.

8. Handling set according to any of claims 1 to 7,
**characterised in that** the second joint (12) has at least two clamping jaws (17) which are positively and/or non-positively in contact with an outer contour of the plug or socket part in the third joint (18) state.

## Revendications

1. Kit d'assistance médicale pour le raccordement de deux implants disposés séparés l'un de l'autre dans l'espace par un conduit de liaison souple (14) prévu le long d'une interface mécanique à la manière d'une liaison connecteur-douille, avec
- un tube (1) et un mandrin (2) introduit dedans, qui prévoit une pointe de mandrin (5) placé fixe de façon amovible par une première liaison de jonction (6) sur la zone de mandrin distale, qui dépasse à l'état assemblé l'extrémité de tube distale,
- un premier profilé de jonction (8) placé sur la zone de mandrin distale, qui fait partie d'une deuxième liaison de jonction (11), ainsi qu'
- un élément adaptateur (7) avec un contre-profilé de jonction (9) constitué en profilé complémentaire au premier profilé de jonction (8) pour constituer la deuxième liaison de jonction (11) ainsi qu'avec un deuxième profilé de liaison (12) constitué en profilé complémentaire de la partie connecteur-douille de l'interface mécanique, qui fait partie d'une troisième liaison de jonction (18).

2. Kit d'assistance selon la revendication 1,
***caractérisé en ce que*** la première liaison de jonction (6) est constituée à la manière d'une liaison filetage-contre-filetage ou à la manière d'une fermeture à baïonnette.

3. Kit d'assistance selon la revendication 1 ou 2,
***caractérisé en ce que*** la deuxième et la troisième liaison de jonction (11, 18) sont basées sur u mécanisme de clipsage et/ou d'encliquetage lors de la jonction desquelles il se produit un bruit acoustiquement perceptible.

4. Kit d'assistance selon l'une quelconque des revendications 1 à 3,
***caractérisé en ce que*** le premier profilé de jonction (8) est constitué en forme de tête et le contre-profilé de jonction de forme concave de telle manière que le premier profilé de jonction (8) peut être assemblé soutenu par une force de pression dans le contre-profilé de jonction concave (9) au cours d'une opération de clipsage acoustiquement perceptible, ou inversement.

5. Kit d'assistance selon l'une quelconque des revendications 1 à 4,
***caractérisé en ce que*** le conduit de liaison souple (14) comporte au moins une extrémité de conduit avec un profilé de jonction constitué sous la forme d'une pièce connecteur-douille et
***en ce que*** le profilé de jonction constitué sous la forme d'une pièce connecteur-douille peut être assemblé dans une position non modifiable dans l'espace par rapport à l'élément adaptateur (7) avec le deuxième profilé de jonction (12) en constituant la troisième liaison de jonction (18).

6. Kit d'assistance selon l'une quelconque des revendications 1 à 5,
***caractérisé en ce que*** l'élément adaptateur (7) ainsi que le profilé de jonction constitué sous la forme d'une pièce connecteur-douille du conduit de liaison souple (14) comportent à l'état assemblé un diamètre de section maximale, qui est plus petit qu'une section attribuée au tube (1) de telle manière que l'élément adaptateur (7) peut être placé axialement, complètement à l'intérieur du tube (1) en constituant la deuxième et la troisième liaison de jonction (11, 18).

7. Kit d'assistance selon l'une quelconque des revendications 1 à 6,
***caractérisé en ce que*** l'élément adaptateur (7) est constitué à la manière d'un cylindre avec un axe de cylindre et deux zones d'extrémité de cylindre opposées le long de l'axe de cylindre,
**en ce que** le contre-profilé de jonction (9) ainsi que le deuxième profilé de jonction (12) de l'élément adaptateur (7) sont respectivement placés aux zones d'extrémité de cylindre opposées.

8. Kit d'assistance selon l'une quelconque des revendications 1 à 7,
***caractérisé en ce que*** le deuxième profilé de jonction (12) comporte au moins deux mâchoires de serrage (17), qui touchent par conformité de forme et/ou de force un profilé extérieur de la partie connecteur-douille à l'état de la troisième liaison de jonction (18).
